# EUROPEAN PATENT SPECIFICATION

(11) **EP 0 798 003 B1**
(45) Date of publication and mention of the grant of the patent: **31.05.2006**
(21) Application number: 97103942.5
(22) Date of filing: 10.03.1997
(51) Int. Cl.: A61L 2/02

(54) **Use of structured depth filters for virus removal**
Verwendung von strukturierten Tiefenfiltern zur Entfernung von Viren
Utilisation de filtres de profondeur pour l'élimination des virus

(30) Priority: 21.03.1996 US 620111
(43) Date of publication of application: 01.10.1997
(73) Proprietor: BAYER CORPORATION, Pittsburgh, PA 15205-9741 (US)
(72) Inventor: Savage, Magaret, Clayton, North Carolina 27520 (US); Hinsken, Werner Dr., 45277 Essen (DE)
(74) Representative: Burkert, Frank

(56) References cited:
- EP-A- 0 679 405
- WO-A-95/18665
- GB-A- 2 045 828
- HOU K. ET AL.: "CAPTURE OF LATEX BEADS, BACTERIA, ENDOTOXIN, AND VIRUSES BY CHARGE-MODIFIED FILTERS" APPLIED AND ENVIRONMENTAL MICROBIOLOGY, vol. 40, no. 5, November 1980, US, pages 892-896, XP002057339

## Description

### BACKGROUND OF THE INVENTION

Field This invention generally relates to removing viruses from solutions and specifically is concerned with the use of structured depth filters for reducing the virus titer of solutions containing biologically active proteins.

Background Various porous filter media are used for the filtration of fine particle size contaminants from liquids. To function as a filter, the media must allow the fluid, commonly water, through while holding back the particulate contaminant. This holding back of the particulate contaminant is accomplished by virtue of the operation, within the porous media, of one or both of two distinctly different filtration mechanisms, namely (1) mechanical straining and (2) electrokinetic particle capture. In mechanical straining, a particle is removed from the fluid stream by physical retention when it attempts to pass through a pore smaller than itself. In the case of the electrokinetic capture mechanism, the particle collides with a surface within the porous material and is retained on the surface by the attractive short range van der Waal's type forces.

Filter media are often described according to their physical form. Some filter media are essentially discrete membranes which function by retaining contaminants upon the surface of the membrane (surface filters). Thus, these filter media primarily operate via mechanical straining, and it is necessary that the pore size of the filter medium be smaller than the particle size of the contaminants that are to be removed from the fluid. Such a filter medium normally exhibits low flow rates and a tendency to clog rapidly.

Other filter media take the form of a porous cake or bed of fine fibrous or particulate material deposited on a porous support or substrate. The solution being filtered must then wend its way through a tortuous path of pores formed in the interstices of the fine material, leaving colloidal contaminants to be retained by the filter material. Because of the deepness of the filter material, the filters are called depth filters (as opposed to surface filters). Depth filters typically retain contaminants by both the siering mechanism and the electrokinetic particle capture mechanism. In the electrokinetic particle capture mode, it is unnecessary that the filter medium have such a small pore size. The ability to achieve the required removal of suspended particulate contaminants with a filter medium of significantly larger pore size is attractive inasmuch as it allows higher flow rates. Furthermore, the filters have a higher capacity to retain particulates, thus having a reduced tendency to clog. See Curling (1980) and Meltzer (1987) for a general discussion of filtration, especially as applied to the pharmaceutical industry.

*Methods of virus inactivation* Many biologically active proteins are used to treat disease conditions in humans. However, due to the sources of the protein (e.g. human plasma or cell expression systems), there is a possibility of viral contamination in the products. It is therefore typical in the manufacture of the protein containing products to incorporate viral reduction or inactivation steps to yield safer products that decrease the risk of viral infection. Reduction in virus titers can be accomplished through either inactivation or removal of the virus.

Methods of inactivation of viruses by treatment with chemicals and/or heat are common in the art. U.S. Pat. No. 4,540,573 to Neurath et al., for example, describes treating protein-containing compositions with trialkylphosphate and detergent to inactivate viruses. U.S. Pat. No. 5,151,499 to Kameyama et al. adds a dry heat step to the trialkylphosphate/detergent process to yield a greater reduction in virus titer. Virus titers may be reduced by virtue of the method used to purify the protein. The Cohn-Oncley process used to purify IgG fractions from blood plasma has been found to yield proteins with reduced viral titers. See, for example, U.S. Pat. No. 4,762,714 to Mitra et al. See also Friedman et al. (1995) for a brief general discussion of reducing the infectivity of blood components.

Several manufacturers have developed products which remove virus from solution via the sieving action of passing the solution through membrane filters with pore sizes smaller than the virus particles. These methods are described in Yuasa et al. (1991), Nader (1994), and DiLeo et al. (1992). The viruses (and their average diameters) which were reported in these references were: hepatitis C virus (30-38 nm), bacteriophage fr (⁻23 nm), φX174 (⁻28 nm), and φ6 bacteriophage (⁻78 nm).

Depth filters are typically used as prefilters to prolong the useful life of the final filter, typically a surface (or membrane) filter. However, depth filters are also known to be useful for retention of viruses, even where the nominal pore size is larger than the average diameter of the virus. McConnell et al. (1984) show a viral clearance of 4 log₁₀ units from water using slow rate sand filtration, a method which uses a "nonstructured" depth filter. See Meltzer (1987), p. 8.

Such high virus clearance has not been reported with "structured" depth filters. Structured depth filters, available preformed into definite sizes and shapes, are prepared by a variety of technologies. Their manufacture comprises the fixed positioning of individual fibers, or bits and pieces of ceramic, metal, or polymer into a shape. This may involve matting, felting, weaving, gluing, heat or solvent sintering, etc. The organic union of fibers or particles that results is the filter element. The interstices among the joined component particles comprise the filter pores. The filtrative action occasioned by these pores involves sieving as well as retention by adsorption.

An example of a structured medium depth filter is given in U.S. Pat. No. 4,859,340 to Hou et al., which describes a filter media sheet comprising fine particulate and a self binding matrix of cellulose fiber, either or both of which are coated with a cationic resin. Related patents are U.S. Pat. Nos. 4,007,113 and 4,007,114.

Jones et al. (1978) describe the use of positively charged depth filters to retain and concentrate poliovirus from water. Payment et al. (1988) describe the use of fiberglass depth filters to recover viruses from water. Both Jones et al. and Payment et al. show greater than 99 % adsorption of virus to the filter.

The surface charge of depth filter media has been shown to play a role in the adsorption and elution of viruses. See Borrego et al. (1991); Sobsey et al. (1984); Jones et al. (1978). For a general discussion of charge modified depth filters, see Mandaro (in Meltzer, 1987, chapter 5). The purpose of these virus recovery assays was generally to recover and concentrate virus from large volumes of solution to improve the sensitivity of virus detection in later assays. The methods were thus generally applied to assaying for the presence or quantity of viral contamination in water treatment. Goyal et al. (1980) have applied the method to recovery of virus from allantoic fluids.

Breitenbach et al. (1995) describe a novel filter material which inactivates viruses and report viral inactivation rates exceeding 5 log₁₀ units. These new filters comprise two different layers of filter material. One layer releases a viricide chemical, in this case iodide, and the other layer removes the viricide as the solution passes through this second filter layer. Although the viral inactivation is substantial, the oxidative character of the viricide may adversely affect the biological activity of the material passing through this filter.

GB-A-2045828 is related to a filter media sheet comprising fine particulate and a self-bonding matrix of cellulose fiber, the surfaces of at least one of which are modified with cationic colloidal silica.

The capture of Latex Beads, Bacteria, Endotoxin and Viruses by Charge-Modified Filters is described in Hou K. et al., applied and Environmental Microbiology, Vol. 40, No. 5, 11/1980, p. 892-896.

Both citations are silent with regard to the present method of reducing viral titer

As noted above, depth filters are currently used in the pharmaceutical industry as prefilters. In other applications they are typically used either as prefilters or as final filters to remove particulate matter from solutions, a process called clarification. Some virus removal is inherent in precipitation steps in the protein purification process; the solutions then may be clarified of residual precipitate via the use of depth filters. However, to our knowledge the use of positively charged, structured depth filters having pore sizes greater than virus sizes to produce substantial reductions in the virus titer of solutions apart from a precipitation step has not been reported.

### SUMMARY OF THE INVENTION

We have now discovered conditions under which use of a structured depth filter for filtration of a solution of biologically active proteins results in a decrease of surprising magnitude in the virus titer. The solution to be filtered should have a pH ranging from about 5.0 to about 7.0, and the filtration should be performed using a cationically charged depth filter which has an average pore diameter greater than the average diameter of the viruses removed. The virus titer in the filtrate is reduced by at least about 3 log₁₀ units or, more preferably, at least about 4 log₁₀ units. The biological activity of the protein is substantially retained: the filtration results in a retention of at least about 80% and more preferably at least about 90% of the original (pre-filtration) activity. The preferred average pore diameter of the filter may range from about 0.1 *µ*m to about 5 *µ*m, with a more preferred range being from about 0.25 *µ*m to about 2 *µ*m. A preferred filter is made from a filter aid such as diatomite and a self binding matrix of cellulose fiber, either or both of which are coated with a cationic resin; a more preferred filter is a ZETA PLUS brand filter marketed commercially by Cuno (Meriden, CT).

### SPECIFIC EMBODIMENTS

This report presents data that evaluate the removal of the several model viruses including Bovine viral diarrhea virus (BVDV), chosen to model for Hepatitis C virus; Pseudorabies virus (PRV), chosen to model for Hepatitis B and the Herpes viruses; and Reo virus type 3 (Reo), chosen to model non-enveloped viruses and for its resistance to physical and chemical inactivation methods.

The data presented show that the depth filters of the examples were capable of reducing virus titers of both enveloped and non-enveloped viruses. Thus, this study provides additional assurance that the solutions treated provide a high margin of safety from the risk of viral transmission.

As used herein, average pore diameter means the size of pore corresponding to the rejection by seiving action of at least about 90% of the particles of that size by the filter.

As used herein, the average diameter of virus means the generally accepted value for the diameter of the virus as reported in the relevant scientific literature.

As used herein, substantial reduction in virus titer means at least about a 3 log₁₀ unit reduction in virus titer.

As used herein, biologically active protein means a protein which demonstrates a measurable function or activity or is useful for an intended result (especially as opposed to the same protein in a denatured or useless state). The function, activity, or intended result could be, e.g., an enzymatic activity or a binding affinity.

As used herein, retaining biological activity means retaining at least about 80% or more preferably at least about 90% of the biological activity.

### Materials and Methods

Effluent III was obtained from pools of human plasma fractionated using the Cohn-Oncley cold ethanol process with modification according to U.S. Pat. No. 4,396,608 to Tenold. The Cohn fractionation process has been generally considered to yield safe immunoglobulin products with respect to potential virus transmission. Each individual unit of plasma used was tested and found nonreactive for hepatitis B surface antigen (HBsAg) and negative for antibodies to Human Immunodeficiency Virus types 1 and 2 (HIV-1, HIV-2), and hepatitis C virus (HCV). Alanine transaminase (ALT), a marker for liver function and by inference damage caused by the hepatitis viruses, did not exceed twice the normal range. Thus, the source material, plasma, was selected and tested to confirm the absence of clinically significant viruses from the Effluent III.

The viruses chosen for this study and their attributes are summarized in Table 1. Bovine viral diarrhea virus (BVDV), an enveloped RNA virus, was selected as the relevant model for Hepatitis C virus. Pseudorabies virus (PRV), an enveloped DNA virus, was chosen as the model for the clinically relevant Herpes viruses: CMV, Epstein-Barr virus and Herpes simplex types 1, 2, 6 and 7. In addition, the non-enveloped RNA virus, Reovirus type 3 (Reo), was chosen as a model virus with resistance to physico-chemical reagents.

**Table 1: Model Viruses Used**

| Attribute | **Virus** | | |
|---|---|---|---|
| | BVDV | Pseudorabies | Reovirus type 3 |
| Strain | NADL (ATCC VR-534) | PRV d1 tk (ATCC VR-2074) or PRV Backer (DuPont/Merck) Wilmington, DE | Abney (ATCC VR-232) |
| Nucleic acid | RNA | DNA | RNA |
| Enveloped | Yes | Yes | No |
| Size | 80-100 nm | 120-220 nm | 60-80 nm |
| Resistance to Physico-chemical agents | Medium | Medium | High |
| Assay Systems | BT cells (ATCC CRL 1390) | RAB-9 (ATCC CRL 1414) | BSC-1 cells (ATCC CCL 26) |

A minimum of two independent runs was conducted for each virus. Viruses were titered in each sample at time 0 to determine the virus load. A solution of 150 mL and 500 mL of Effluent III spiked with model virus was passed through a ZETA PLUS ZP04701-50CP depth filter (Cuno, Inc., Meriden, CT). One additional filtration was performed spiking with Reo which used 650 mL to maximize the volume to surface area for the filtration. The Effluent III was spiked at 1 part virus plus 19 parts of the starting material for the 150 mL experiments and for the additional 650 mL Reo experiment (to increase the Reo virus load and thus provide a test of the robustness of the filtration) and 1 part virus plus 99 parts of the starting material for the 500 mL experiments to conserve virus.
Virus Quantitation Assays Standard cell culture conditions were used for virus infectivity assays with RPMI 1640 or Eagle minimal essential medium (MEM) supplemented with non-essential amino acids, HEPES buffer (N-[2-Hydroxyethyl]piperazine-N'[2-ethane sulfonic acid]), fetal bovine serum and antibiotics. The RPMI 1640 medium was used for the MT-4 cells only. Virus titers were quantitated by tissue culture infectious dose assays at 50% infectivity (TCID₅₀). TCID₅₀ titers were calculated by the method of Spearman-Karber. See Cavalli-Sforza (1974), p. 171-173.

Ninety-six well micro titer plates were used with the appropriate confluent cells (BVDV in Bovine Turbinate (BT) cells, PRV in Rab-9 cells, Reo in BSC-1 or Vero Cells). The Becker strain of PRV was used for studies where a higher titer was needed. The virus-spiked process inoculum was 0.1 mL per well. Log₁₀ or half-log₁₀ dilutions were made in HBSS from 10^{-0.5} or 10⁻¹ through 10⁻⁶, 4 replicate wells per dilution for one of the fractionation runs. For the second run, an additional 8 replicate wells per dilution were used for a total of 12 wells. This was performed to confirm the sensitivity of the assays using 4 replicate wells per dilution. Three experiments were performed for the filtrations with BVDV and PRV. Four experiments were performed spiking with Reo. Half-log dilutions were made in HBSS from 10^{-0.5} to 10^{-7.5} with 12 replicate wells per dilution.

The test material was allowed to contact the cells for 1 hour at 37°C to allow for virus adsorption. The material was removed and fresh MEM added to the wells. MEM was used for PRV and Reo. For BVDV, the MEM was supplemented with equine serum rather than fetal bovine serum. The plates were incubated for 5 to 7 days at 36 ± 2°C. After incubation, cell monolayers were observed microscopically for the presence of viral infection as indicated by cytopathic effect (CPE). After 5 to 7 days, CPE induced by BVDV and PRV could easily be discerned by microscopic examination. However, if determination of CPE was difficult due to cell overgrowth, Reo infected cells were stained to enhance visualization of CPE. The cells were fixed for 10 minutes with cold 95 % ethanol (EtOH), then the EtOH removed and cells stained with May-Grunwald-Giemsa or Crystal Violet stain. After staining and washing with distilled water, the cells were examined microscopically. Uninfected cells appeared as dark blue monolayers while foci of virus infected cells appeared as very lightly stained areas.

### Tests for cytotoxicity and viral interference

For those fractionation product samples expected to contain low levels of virus, undiluted samples without virus were applied to each of the indicator cell lines to assess the matrix effect (ethanol, process buffers and proteins, etc.) on the cells.

Effluent III was inoculated onto BT and Vero cells undiluted and at 1:10 in HBSS. Filtrate III was inoculated onto Rab 9 and BSC-1 cells undiluted at 1:3.2 in HBSS. HBSS was used as the control. An additional control included HBSS containing 17% ethanol to assess effects of ethanol concentration on cells. After the 1 hour exposure to the material, the effluent was removed and cells were fed with fresh MEM. Cells were examined after 7 days for cytotoxicity.

Viral interference assays were conducted by diluting virus stocks in HBSS as the control and in Effluent III diluted 1:10 in HBSS. The two viruses used for these experiments were BVDV and Reo. Log₁₀ dilutions were made and cells were inoculated with 0.1 mL, for 1 hour adsorption time. The material was removed and media was added. At 7 days post infection, cells were examined microscopically for viral CPE.

Viral interference assays for Filtrate III were conducted by diluting virus stocks in HBSS as the control and in Filtrate III diluted 1:3.2 in HBSS. Half-log₁₀ dilutions were made and cells were inoculated and incubated as described above. The viruses used were PRV and Reo.

The effectiveness of the process steps to clear the relevant model viruses was determined by comparing the virus load in the input to that of the output to calculate the reduction factor. The majority of the 95 % confidence intervals for virus titrations using log₁₀ dilutions were approximately 1 log₁₀. When half-log dilutions were used, the 95% confidence intervals for the virus titrations were approximately 0.3 - 0.4 log₁₀.

The invention is demonstrated further by the following illustrative examples.

### EXAMPLE 1

### Filtration of Effluent III to Filtrate III

Results of virus clearance experiments with the three model viruses are indicated in Table 2. For a given experiment, the virus in Minimal Essential Medium (MEM) was added to the given volume of Effluent III (as described above). For example, to 504 mL of Effluent III, 5 g of Reovirus in MEM was added. The pH of the protein containing Effluent III plus virus was adjusted to 5.4 and chilled to -5° C. Filter Aid was added to a final concentration of 0.6% . A 10 mL sample was removed for initial virus titration. The remaining solution was filtered through a CUNO ZETA PLUS® ZP04701- 50 CP filter at a pressure of 10 psi. The resulting filtrate was collected and samples were titered for detection of virus. After completion of filtration, the filter was removed and placed in 50 mL of balanced salt solution and shaken for 15 minutes. (Filters from smaller volume experiments were shaken in correspondingly smaller volumes of balanced salt solution.) A sample of this was titered for detection of virus retained by the filter.

The results in Table 2 are expressed as the log ₁₀ TCID₅₀/mL in each sample. The average log₁₀ reduction factor was calculated by multiplying the log₁₀ TCID₅₀/mL by the volume of the Effluent III to determine the total virus input load for each of the viruses [(vol) (log₁₀ TCID₅₀/mL)] and then dividing by the total virus remaining in the Filtrate III [(vol) (log₁₀ TCID₅₀/mL)]. The average log₁₀ reduction factor was ≤ 1 for BVDV, ≥3.8 for PRV, and ≥4.5 for Reo. Results reported as ≥ indicates complete clearance of the input load of virus was achieved. Higher clearance or inactivation factors could be achieved if it were possible to obtain higher titers of virus for the input spike.

**Table 2: Depth Filtration of Effluent III to Filtrate III Virus in Fractionation Samples (Log₁₀ TCID₅₀/mL)**

| | BVDV | | | PRV | | | Reovirus | | | |
|---|---|---|---|---|---|---|---|---|---|---|
| | A^{a} | B | C^{b} | A | B | C | A | B | C | D^{c} |
| HBSS | 4.4 | 5.8 | 5.3 | 5.8 | 5.8 | 5.3 | 6.5 | 6.7 | 6.3 | 7.0 |
| Effluent III | 7.0^{d} | 7.7 | 7.7 | 5.4 | 5.3 | 4.7 | 5.3 | 5.8 | 5.6 | 6.6 |
| Filtrate | 5.6 | 6.9 | 7.3 | ≤1.3 | ≤1.3 | ≤1.3 | ≤1.3 | ≤1.3 | ≤1.3 | ≤1.3 |
| Filter pad Retentate^{e} | 7.1 | 7.7 | 7.6 | 6.8 | 6.9 | 6.1 | 7.1 | 7.2 | 6.4 | 7.4 |
| Log₁₀ Reduction | 1.4 | 0.8 | 0.4 | ≥4.1 | ≥4.0 | ≥3.4 | ≥4.0 | ≥4.5 | ≥4.3 | ≥5.3 |
| Average Log₁₀ Reduction | 0.9 | | | ≥3.8 | | | ≥4.5 | | | |

| | | | | | | | | | | |
|---|---|---|---|---|---|---|---|---|---|---|
| ^{a} A and B = Two replicate experiments - 150 mL filtration volume | | | | | | | | | | |
| ^{b} C = 500 mL filtration volume | | | | | | | | | | |
| ^{c} D = For Reo 650 mL filtration volume | | | | | | | | | | |
| ^{d} Total virus (Total log₁₀ TCID₅₀) used for BVDV because virus was found in all samples of Filtrate III | | | | | | | | | | |
| ^{ee}Filter pads were suspended in 12-18 mL of HBSS, then a sample removed for filtration | | | | | | | | | | |

It is thought that the BVDV was not retained by the depth filter because this virus may have a much lower charge density ratio than the other viruses used. The presence of proteins other than IgG which were removed from the Effluent III by the depth filter may have competed with this virus such that the virus was not retained. In other experiments where the contaminating proteins were not present and only IgG was present in the protein solution, the BVDV was retained by the depth filter.

### EXAMPLE 2

Virus clearance experiments were performed as described in Example 1 on three more viruses: hepatitis A virus, bovine parvovirus, and porcine parvovirus. Results are presented in Table 3. Clearance rates were ≥4.0 log₁₀ units for hepatitis A virus, ≥2.7 log₁₀ units for bovine parvovirus, and ≥4.7 log₁₀ units for porcine parvovirus. It is thought with further refinement of conditions that the virus clearance for bovine parvovirus would be ≥3.0 log₁₀ units as well.

**Table 3: Depth Filtration of Effluent III to Filtrate III Virus in Fractionation Samples (Log₁₀ TCID₅₀/mL)**

| | Hepatitis A Virus | | | | Bovine Parvovirus | | | Porcine Parvovirus | |
|---|---|---|---|---|---|---|---|---|---|
| | A^{a} | B^{b} | C^{a} | D^{a} | A^{a} | B^{a} | C^{b} | A^{c} | B^{a} |
| HBSS | 6.1 | 4.8 | 6.7 | 5.6 | 5.0 | 5.0 | 5.2 | 6.2 | 6.5 |
| Effluent III | 5.6 | 4.0 | 6.2 | 5.5 | 4.0 | 3.8 | 4.3 | 5.8 | 6.1 |
| FiltrateIII | ≤1.3 | ≤1.3 | 1.6 | ≤1.3 | ≤1.3 | ≤1.3 | ≤1.3 | ≤1.3 | ≤1.3 |
| Filter pad Retentate | 6.1 | 5.2 | 6.8 | 6.2 | 4.2 | 4.5 | 5.1 | 7.1 | 6.3 |
| Log₁₀ Reduction | ≥4.3 | ≥2.7 | 4.6 | ≥4.2 | ≥2.7 | ≥2.5 | ≥3.0 | ≥4.5 | ≥4.8 |
| Average Log₁₀ Reduction | ≥4.0 | | | | ≥2.7 | | | ≥4.7 | |

| | | | | | | | | | |
|---|---|---|---|---|---|---|---|---|---|
| ^{a} 150 mL filtration volume | | | | | | | | | |
| ^{b}500 mL filtration volume | | | | | | | | | |
| ^{c} 600 mL filtration volume | | | | | | | | | |

Based on our experience in monitoring the activity of commercially produced immune globulins, the above described conditions would not be expected to adversely affect the biological activity of the proteins and there would be a substantial retention of the original biological activity of the protein.

The above examples are intended to illustrate the invention and it is thought variations will occur to those skilled in the art. Accordingly, it is intended that the scope of the invention should be limited only by the claims below.

### REFERENCES

U.S. Pat. No. 4,007,113 to Ostreicher, E. A., issued Feb. 8, 1977.
U.S. Pat. No. 4,007,114 to Ostreicher, E. A., issued Feb. 8, 1977.
U.S. Pat. No. 4,396,608 to Tenold, R. A., issued Aug. 2, 1983.
U.S. Pat. No. 4,540,573 to Neurath, A. R., et al., issued Sept. 10, 1985.
U.S. Pat. No. 4,762,714 to Mitra, G., et al., issued Aug. 9, 1988.
U.S. Pat. No. 4,859,340 to Hou, K. C., et al., issued Aug. 22, 1989.
U.S. Pat. No. 5,151,499 to Kameyama, S., et al., issued Sept. 29, 1992.

Borrego, J. J., et al., "Development and Application of new Positively Charged Filters for the Recovery of Bacteriophages from Water," *Appl. Environ. Microbiol. 57:* 1218-22 (1991).
Breitenbach, J., et al., German Pat. Application No. DE 43 43 226 A1, published June 22, 1995.
Cavalli-Sforza, L., Biometrie Grunzuge Biologisch-medizinischer Statistik [Biometry, the Basics of Biological and Medical Statistics], Gustav Fischer Verlag (Stuttgart, 1974).
Curling, J. M., ed., Methods of Plasma Protein Fractionation, Academic Press (London, 1980).
DiLeo, A. J., et al., "High Resolution Removal of Virus from Protein Solutions Using a Membrane of Unique Structure," *Bio*/*Technol.,* **10**: 182-188 (1992).
Friedman, L. J., et al., "Reducing the Infectivity of Blood Components - What We Have Learned," *Immunolog. Invest.* **24**: 49-71 (1995).
Goyal, S. M., et al., "Simple Method for the Concentration of Influenza from Allantoic Fluid on Microporous Filters," *Appl. Environ. Microbiol.* **39**: 500-504 (1980).
Jones, B. L., et al., "Concentration of Poliovirus from Tap Water Using Positively Charged Microporous Filters," Presented at Ann. Conf. Amer. Water Works Ass'n, Atlantic City, NJ, June 26-30, 1978.
McConnell, L. K., et al., "Reovirus Removal and Inactivation by Slow Rate Sand Filtration," *Appl. Environ. Microbiol.,* **48**: 818-825 (1984).
Meltzer, T.H., Filtration in the Pharmaceutical Industry, Marcel Dekker (New York, 1987).
Nader, W., "Validation of a UF System for the Removal of Viruses and Scrapie Agent," *Genet. Engin. News,* Nov, 1, 1994, p. 16.
Payment, P., et al., "Wound Fiberglass Depth Filters as a Less Expensive Approach for the Concentration of Viruses from Water," *Can. J. Microbiol.,* **34**: 271-272 (1988).
Sobsey, M. D., et al., "Influence of Water Quality on Enteric Virus Concentration by Microporous Filter Methods," *Appl. Environ. Microbiol.,* **47:** 956-960 (1984).
Yuasa, T., et al., "The Particle Size of Hepatitis C Virus Estimated by Filtration Through Microporous Regenerated Cellulose Fiber," *J. Gen. Viral.,* **72**: 2021-2024 (1991).

## Claims

1. A method of reducing the titer of viruses in an aqueous solution of biologically active plasma proteins without adversely affecting the activity of the protein, the method comprising the step of passing the solution at a pH ranging from about 5.0 to about 7.0 through a structured depth filter comprising a positively charged porous filter element, the average pore diameter of the filter element being larger than the average diameter of the viruses and the filtration being done under conditions resulting in a virus titer reduction of at least about 3 log₁₀ units in the filtered solution while retaining at least about 80% of the biological activity of the proteins, the viruses being selected from the group consisting of hepatitis A virus, pseudorabies virus, herpes simplex virus type 1, herpes simplex virus type 2, herpes simplex virus type 6, herpes simplex virus type 7, cytomegalovirus, Epstein-Barr virus, reovirus, and porcine parvovirus.

2. The method of claim 1 wherein the average pore diameter of the filter element is within the range of about 0.25 µm to about 2 µm.

3. The method of claim 1 or 2 wherein the filter element comprises a filter aid and a self binding matrix of cellulose fiber, at least one of which is coated with a cationic resin.

4. The method of claim 3 wherein the filter aid is diatomite.

5. The method of claim 3 wherein the proteins are IgG.

## Patentansprüche

1. Verfahren zur Herabsetzung des Titers von Viren in einer wässrigen Lösung biologisch aktiver Plasmaproteine, ohne die Wirksamkeit des Proteins nachteilig zu beeinflussen, wobei das Verfahren die Stufe umfasst, in der man die Lösung bei einem pH-Wert von ca. 5,0 bis ca. 7,0 durch ein strukturiertes Tiefenfilter aus einem positiv geladenen porösen Filterelement, dessen Durchschnittsporendurchmesser größer als der Durchschnittsdurchmesser der Viren ist, laufen lässt und die Filtration unter Bedingungen durchführt, um eine Herabsetzung des Virus-Titer um mindestens ca. 3 log₁₀-Einheiten in der filtrierten Lösung unter Beibehaltung von mindestens ca. 80 % der biologischen Aktivität der Proteine zu ergeben, wobei die Viren aus der Gruppe ausgewählt sind, bestehend aus Hepatitis A-Virus, Pseudorabies-Virus, Herpes simplex-Virus-Typ 1, Herpes simplex-Virus-Typ 2, Herpes simplex-Virus-Typ 6, Herpes simplex-Virus-Typ 7, Zytomegalovirus, Epstein-Barr-Virus, Reovirus und aus Schweine-Parvovirus.

2. Verfahren gemäß Anspruch 1, worin der Durchschnittsporendurchmesser des Filterelements im Bereich von ca. 0,25 bis ca. 2 µm liegt.

3. Verfahren gemäß Anspruch 1 oder 2, worin das Filterelement eine Filterhilfe und eine selbst-bindende Matrix aus Cellulosefaser umfasst, von denen mindestens ein Bestandteil mit einem kationischen Harz überzogen ist.

4. Verfahren gemäß Anspruch 3, worin die Filterhilfe Diatomit ist.

5. Verfahren gemäß Anspruch 3, worin die Proteine IgG sind.

## Revendications

1. Procédé pour réduire le titre en virus d'une solution aqueuse de protéines plasmatiques biologiquement, actives sans effet néfaste sur l'activité de la protéine, procédé comprenant l'étape consistant à faire passer la solution à un pH d'environ 5,0 à environ 7,0 à travers un filtre à profondeur structurée comprenant un élément filtrant poreux chargé positivement, le diamètre des pores de l'élément filtrant étant supérieur au diamètre moyen des virus et la filtration étant effectuée dans des conditions ayant pour résultat une réduction du titre en virus d'au moins environ 3 unités log₁₀ dans la solution filtrée tout en conservant au moins environ 80 % de l'activité biologique des protéines, les virus étant choisis dans le groupe consistant en le virus de l'hépatite A, le virus pseudorabique, le virus Herpes simplex de type 1, le virus Herpes simplex de type 2, le virus Herpes simplex de type 6, le virus Herpes simplex de type 7, le cytomégalovirus, le virus d'Epstein-Barr, un réovirus et le parvovirus porcin.

2. Procédé suivant la revendication 1, dans lequel le diamètre moyen des pores de l'élément filtrant est d'environ 0,25 µm à environ 2 µm.

3. Procédé suivant la revendication 1 ou 2, dans lequel l'élément filtrant comprend un adjuvant de filtration et une matrice à liaison autonome de fibres de cellulose, au moins un de ces constituants étant revêtu d'une résine cationique.

4. Procédé suivant la revendication 3, dans lequel l'adjuvant de filtration est la diatomite.

5. Procédé suivant la revendication 3, dans lequel les protéines sont des IgG.
